# EUROPEAN PATENT SPECIFICATION

(11) **EP 4 575 867 B1**
(45) Date of publication and mention of the grant of the patent: **28.10.2026**
(21) Application number: 22955684.0
(22) Date of filing: 17.08.2022
(51) Int. Cl.: H04L 9/00, G06F 21/64, H04L 9/32

(54) **INFORMATION PROCESSING SYSTEM, INFORMATION PROCESSING METHOD, AND PROGRAM**
INFORMATIONSVERARBEITUNGSSYSTEM, INFORMATIONSVERARBEITUNGSVERFAHREN UND PROGRAMM
SYSTÈME DE TRAITEMENT D'INFORMATIONS, PROCÉDÉ DE TRAITEMENT D'INFORMATIONS ET PROGRAMME

(43) Date of publication of application: 25.06.2025
(73) Proprietor: NEC Corporation, 108-8001 Tokyo (JP)
(72) Inventor: TAKAHASHI Shingo, Tokyo 108-8001 (JP); TANAKA Yuki, Tokyo 108-8001 (JP); ONOUE Kousuke, Tokyo 108-8001 (JP)
(74) Representative: Betten & Resch
(86) International application number: PCT/JP2022/031011
(87) International publication number: WO 2024/038510

(56) References cited:
- WO-A1-2022/054869
- JP-A- 2017 204 704
- JP-A- 2019 139 750
- JP-A- 2019 185 642
- JP-A- 2022 042 765
- US-A1- 2018 218 779
- MOHAMMED ALGHAZWI ET AL: "Blockchain for Genomics: A Systematic Literature Review", ARXIV.ORG, CORNELL UNIVERSITY LIBRARY, 201 OLIN LIBRARY CORNELL UNIVERSITY ITHACA, NY 14853, 19 November 2021 (2021-11-19), XP091100670

## Description

### Technical Field

The present invention relates to an information processing system, an information processing method, and a program.

### Background Art

In the cancer medical treatment so far, treatments and drugs have been selected for each type of cancer such as lung cancer, colorectal cancer, and breast cancer.
However, in the 2000s, elucidation of molecules (proteins) that cause cancer and genetic mutations that are bases of the molecules has progressed, and "molecular target drugs" acting on such molecules, genes, and the like can be used for treatment. Beyond development of molecular target drugs, treatment methods targeting molecules and genetic mutations for individual patients are also being developed. Here, performing a treatment suitable for each person according to not only the type of cancer but also characteristics of the cancer such as a genetic mutation is referred to as "personalized treatment". Conventionally, "personalized treatment" based on cancer gene information has been performed mainly on the basis of "cancer gene examination" for examining a small number of genes and "cancer gene panel examination" for examining a large number of genes simultaneously.

In recent years, treatments (hereinafter, referred to as cancer vaccine treatments) using cancer immunotherapy that "attack and eliminate cancer cells by the action of the immune system inherent in humans" have further progressed. A "peptide vaccine" is known as one of cancer vaccine treatments. The peptide vaccines contain antigens that are markers of cancer. The mechanism that, when a peptide containing this antigen is directly injected into the body, a natural immune function inherent in humans detects an abnormality and attacks the antigen that is a marker of cancer as a target to kill cancer cells.

In recent years, an era has been reached in which genetic mutations of cancers of respective patients can be comprehensively and simply ascertained, and the importance of "peptides" newly generated by genetic mutations has been highlighted in cancer immunotherapy. This peptide is a mutated peptide called a "neoantigen". It has been revealed that when the mutated peptide becomes attached to human leukocyte antigen (HLA) and appears on the surface of cancer cells, cytotoxic T lymphocytes (CTLs) regard the cancer cells as an enemy and can kill the cancer cells because the mutated peptide are not peptides on the surface of normal cells. Currently, clinical trials are being conducted in Europe and the United States to confirm whether the personalized cancer vaccine therapy using "neoantigens" is effective as a cancer treatment method or recurrence prevention method. In addition, Patent Literature 1 discloses a method for identifying a fragment of a polypeptide that is immunogenic to a specific human subject and a method for preparing a personalized pharmaceutical composition containing the polypeptide fragment.

Patent Literature 2 discloses a blockchain-based system for securely storing and retrieving clinical data associated with a clinical trial. When the data is stored in a data store, a signed hash of the data is additionally stored in a blockchain. This allows recipients of the clinical data to subsequently verify the provenance and the integrity of the data retrieved from the data store.

### Citation List

### Patent Literature

Patent Literature 1: JP 2020-510698 A
Patent Literature 2: US 2018/218779 A1.

### Summary of Invention

### Technical Problem

Since the type and number of mutated peptides called "neoantigens" vary depending on the individual patient, it is necessary to produce a peptide vaccine individually for each patient from genetic mutations of cancer tissues of the patient. Here, in the case of a personalized treatment (for example, personalized cancer vaccine therapy), there are a step in which a gene analysis organization (for example, a sequence vendor) analyzes a gene sequence of a specimen of a patient, a step in which a recipe creation organization creates a recipe of a drug (for example, a cancer vaccine) to the patient from a gene analysis result, and a step in which a drug manufacturing organization (for example, a pharmaceutical company) manufactures a drug (for example, a cancer vaccine) from the recipe.

As described above, in a distribution process in creating a personalized drug (for example, a cancer vaccine), authenticity of gene sequence data (also referred to as genome information) of a patient's specimen or vaccine data is required.

In addition, authenticity is required for a specification of a drug dedicated to the target patient (for example, personalized vaccine) and a contract of a drug dedicated to the target patient (for example, personalized vaccine).

In addition, there is a demand for a mechanism for ensuring authenticity for a manufacturing report of a drug dedicated to the target patient (for example, personalized vaccine), a manufacturing test report of a drug dedicated to the target patient (for example, personalized vaccine), a delivery document of a drug dedicated to the target patient (for example, personalized vaccine), a receipt of a drug dedicated to the target patient (for example, personalized vaccine), or the like.

As described above, there is a demand for a mechanism for ensuring authenticity of target content (for example, genome data, recipe data, and the like) in a distribution process in creating a drug dedicated to the target patient (for example, personalized vaccine). Here, the target content is genome data of the target patient, recipe data for a drug dedicated to the target patient (for example, personalized vaccine), a specification of the drug dedicated to the target patient (for example, personalized vaccine), a contract of the drug dedicated to the target patient (for example, personalized vaccine), a manufacturing report of the drug dedicated to the target patient (for example, personalized vaccine), a manufacturing test report of the drug dedicated to the target patient (for example, personalized vaccine), a delivery document of the drug dedicated to the target patient (for example, personalized vaccine), a receipt of the drug dedicated to the target patient (for example, personalized vaccine), or the like.

Not only cancer vaccines but also autoimmune diseases may be treated similarly, and there is a similar problem in personalized drugs for autoimmune diseases.

The present embodiment has been made in view of the above problems, and an object thereof is to provide an information processing system, an information processing method, and a program capable of securing authenticity of target content in a distribution process in creation of a personalized drug.

### Solution to Problem

An information processing system according to a first aspect of the present invention is an information processing system including: a first terminal; a second terminal; and a management system, in which the first terminal outputs a first hash value from data including at least target content, and transmits first verification data including either the first hash value or information based on the first hash value, the management system records the first verification data received from the first terminal in a blockchain, the management system transmits the first verification data recorded in the blockchain to the second terminal, and the second terminal acquires a first hash value from the first verification data received from the management system, acquires the target content and generates a second hash value from the acquired target content, and determines whether the first hash value matches the second hash value.

An information processing system according to a second aspect of the present invention is the information processing system according to the first aspect, in which the first terminal is used by a gene analysis organization that generates genome information of a target patient from a specimen of the target patient, the second terminal is used by a recipe creation organization that creates a vaccine recipe, and the target content is genome information of the target patient.

An information processing system according to a third aspect of the present invention is the information processing system according to the second aspect, in which the genome information of the target patient includes gene data of cancer cells of the target patient and gene data of normal cells of the target patient.

An information processing system according to a fourth aspect of the present invention is the information processing system according to the first aspect, in which the first terminal is used by a recipe creation organization that creates a vaccine recipe, the second terminal is used by a drug manufacturing organization that manufactures a personalized drug from the vaccine recipe, and the target content is a vaccine recipe for a target patient.

An information processing system according to a fifth aspect of the present invention is the information processing system according to any one of the first to fourth aspects, in which the information based on the first hash value is obtained by encrypting the first hash value with a first private key, when transmitting the first verification data, the first terminal transmits the first hash value encrypted with the first private key, and when acquiring the first hash value included in the first verification data, the second terminal acquires the first hash value by decrypting the first verification data with a first public key corresponding to the first private key.

An information processing system according to a sixth aspect of the present invention is the information processing system according to any one of the first to fifth aspects, in which the first verification data received from the first terminal recorded in the blockchain includes drug identification information for identifying the personalized drug for the target patient, in a case where the first hash value matches the second hash value, the second terminal transmits second verification data including the drug identification information and the first hash value or the second hash value, and the management system records the second verification data received from the second terminal in the blockchain.

An information processing system according to a seventh aspect of the present invention is the information processing system according to the sixth aspect, further including an authentication system, in which the authentication system searches for a block in a blockchain using target drug identification information as a key, and in a case where hash values included in two obtained blocks match as a result of the search, determines that the transfer of the target content has been performed normally.

An information processing system according to an eighth aspect of the present invention is an information processing system according to the second or third aspect, the information processing system further including a third terminal used by a drug manufacturing organization that manufactures a personalized drug from a vaccine recipe, in which the second terminal outputs a hash value from data including at least a recipe of a drug for a target patient, and transmits third verification data including either the hash value or information based on the hash value, the management system records the third verification data received from the second terminal in a blockchain, the management system transmits the third verification data recorded in the blockchain to the third terminal, and the third terminal acquires a fourth hash value included in the third verification data received from the management system, acquires the recipe and generates a fourth hash value from the acquired recipe, and determines whether the third hash value matches the fourth hash value.

An information processing system according to a ninth aspect of the present invention is the information processing system according to the eighth aspect, in which when transmitting the third verification data, the second terminal transmits the third hash value encrypted with a second private key, and when acquiring the third hash value included in the third verification data, the third terminal acquires the second hash value by decrypting the third verification data with a second public key corresponding to the second private key.

An information processing system according to a tenth aspect of the present invention is the information processing system according to the eighth or ninth aspect, in which the third verification data received from the second terminal recorded in the blockchain includes drug identification information for identifying a personalized drug for a target patient,
in a case where the third hash value matches the fourth hash value, the third terminal transmits fourth verification data including the drug identification information and the third hash value or the fourth hash value, and
the management system records the fourth verification data received from the third terminal in the blockchain.

An information processing system according to an eleventh aspect of the present invention is an information processing system according to any one of the first to tenth aspects, the information processing system further including: a fourth terminal used by a medical institution; and a fifth device used by a distribution company, in which the fourth terminal transmits fifth verification data for proving that a specimen of a target patient has been delivered from the medical institution to the distribution company, the management system records the fifth verification data received from the fourth terminal in a blockchain, the fifth terminal transmits sixth verification data for proving that the distribution company has received a specimen of a target patient from the medical institution, and the management system records the sixth verification data received from the fifth terminal in a blockchain.

An information processing system according to a twelfth aspect of the present invention is the information processing system according to the eleventh aspect, in which the fifth verification data includes a hash value of the document data of the personalized drug encrypted with a third private key for a medical institution, and the sixth verification data includes a hash value of the document data of the personalized drug encrypted with a fourth private key for a distribution company.

An information processing method according to a thirteenth aspect of the present invention includes the steps of: outputting, by a first terminal, a first hash value from data including at least target content, and transmitting, by a management system, first verification data including either the first hash value or information based on the first hash value; recording, by the management system, the first verification data received from the first terminal in a blockchain; transmitting, by the management system, the first verification data recorded in the blockchain to the second terminal; and acquiring, by the second terminal, a first hash value from the first verification data received from the management system, acquiring the target content and generating a second hash value from the target content, and determining whether the first hash value matches the second hash value.

A program according to a fourteenth aspect of the present invention is a program for causing a computer to execute: a procedure of acquiring a first hash value from first verification data recorded in a blockchain, the first verification data including either a first hash value generated from data including at least target content or information based on the first hash value; a procedure of generating a second hash value from the target content; and a procedure of determining whether the first hash value matches the second hash value.

### Advantageous Effects of Invention

According to one aspect of the present invention, when a first hash value recorded in the blockchain matches a second hash value generated from target content, it is ensured that the first hash value and the second hash value are the same target content. Therefore, authenticity of the target content can be ensured.

### Brief Description of Drawings

Fig. 1 illustrates a configuration of an information processing system according to the present embodiment.
Fig. 2 is a schematic configuration diagram of a terminal according to the present embodiment.
Fig. 3 is a schematic configuration diagram of a management system according to the present embodiment.
**Fig.** 4 is a schematic configuration diagram of an authentication system according to an embodiment.
Fig. 5 is a schematic diagram illustrating an example of an outline of processing of a personalized drug creating process.
Fig. 6 is a schematic diagram illustrating an example of a flow of transferring of a file of target content.

### Description of Embodiments

Hereinafter, each embodiment will be described with reference to the drawings. However, unnecessarily detailed description may be omitted. For example, a detailed description of a well-known matter and a repeated description of substantially the same configuration may be omitted. This is to avoid unnecessary redundancy of the following description and to facilitate understanding of those skilled in the art.

In addition, in a distribution process in creating a personalized drug (for example, a cancer vaccine), a mechanism for ensuring that a gene analysis organization that has generated data is a creation party is required for gene sequence data (also referred to as genome data) of a patient's specimen. Similarly, in a distribution process in creating a personalized drug (for example, a cancer vaccine), there is a demand for a mechanism for ensuring that a recipe creation organization that has created a vaccine recipe is a creation party for vaccine recipe data.

Similarly, regarding a specification of a drug dedicated to the target patient (for example, personalized vaccine) and a contract of a drug dedicated to the target patient (for example, personalized vaccine), there is a demand for a mechanism for ensuring that a creation organization that created them is a creation party.

Similarly, regarding a manufacturing report of a drug dedicated to the target patient (for example, personalized vaccine), a manufacturing test report of a drug dedicated to the target patient (for example, personalized vaccine), a delivery document of a drug dedicated to the target patient (for example, personalized vaccine), a receipt of a drug dedicated to the target patient (for example, personalized vaccine), and the like, there is a demand for a mechanism for ensuring that a creation organization that created them is a creation party.

As described above, in addition to or in place of the above problem, there is a demand for a mechanism for securing authenticity of the creation party who created the target content in the distribution process in the creation of the personalized drug.

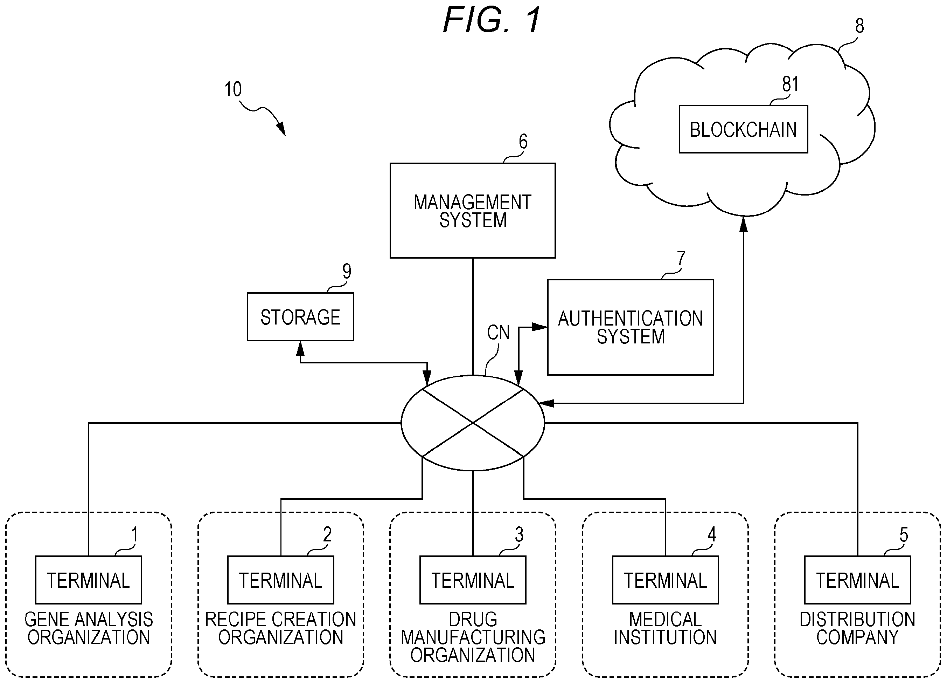

Fig. 1 illustrates a configuration of an information processing system according to the present embodiment. As illustrated in Fig. 1, the information processing system 10 includes a terminal 1 used by a gene analysis organization, a terminal 2 used by a recipe creation organization, a terminal 3 used by a drug manufacturing organization, a terminal 4 used by a medical institution, and a terminal 5 used by a distribution company.

Here, the gene analysis organization generates genome information (also referred to as gene data) of the target patient from a specimen of the target patient. The specimen of the target patient includes, for example, cancer tissue and normal tissue of the same target patient, and in this case, the generated genome information of the target patient is gene data (for example, DNA data) of cancer cells of the target patient and gene data (for example, DNA data) of normal cells of the target patient. The recipe creation organization creates a vaccine recipe.

The information processing system 10 further includes a management system 6, an authentication system 7, a distributed network 8 for a blockchain, and a storage 9. Each of the terminals 1 to 5, the authentication system 7, and the storage 9 is communicably connected to the management system 6 via a communication network CN. Here, the terminals 1 to 5 are, for example, a computer such as a smartphone, a tablet terminal, a notebook computer, or a personal computer. Each of the terminals 1 to 5 includes at least one processor and at least one memory, and stores a unique private key. The authentication system 7 stores each of public keys corresponding to the private keys held by each of the terminals 1 to 5. Data (for example, gene data, vaccine recipe data, and the like) of the above target content is stored in the storage 9.

Note that the distributed network 8 includes a plurality of computers (not illustrated), and these terminals are communicably connected by a peer to peer (P2P) method as an example. Here, the P2P method is a connection method in which a plurality of equivalent computers perform direct communication on a one-to-one basis. A blockchain 81 is stored in each of a plurality of computers included in the distributed network 8. In the blockchain 81, information is stored by blockchain technology, and for example, a history of delivery in a distribution process in the creation of the personalized drug is stored.

Since the configuration of the terminals 1 to 5 is common, the configuration of the terminal 1 will be described below as a representative of the terminals 1 to 5.

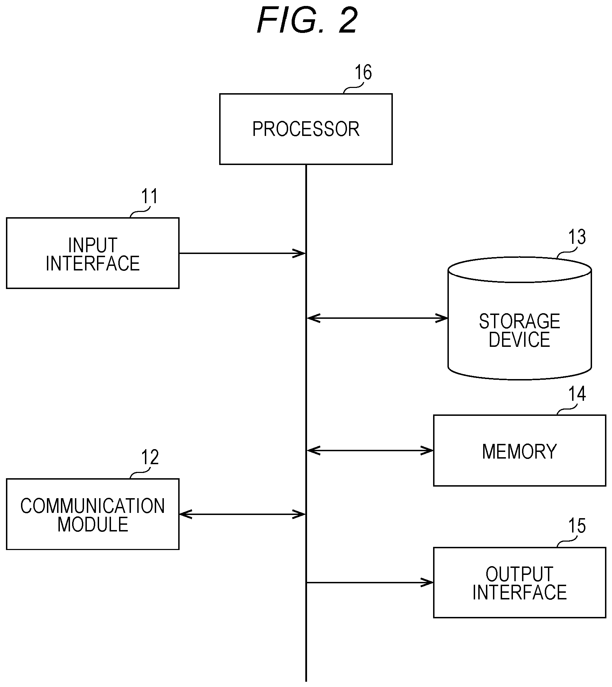

Fig. 2 is a schematic structural diagram of a terminal in the present embodiment. As shown in Fig. 2, the terminal 1 includes an input interface 11, a communication module 12, a storage device 13, a memory 14, an output interface 15, and a processor 16. Note that, here, as one aspect, the terminal 1 will be described as including one processor 16, but there may be a plurality of processors, that is, one or more processors may be included. Furthermore, here, as one aspect, the terminal 1 will be described as including one storage device 13, but there may be a plurality of storage devices, that is, one or more storage devices may be included.

The input interface 11 receives an input from, for example, a person in charge of the gene analysis organization, and outputs an input signal corresponding to the received input to the processor 16.

The communication module 12 is connected to the communication network CN and communicates with the management system 6 and the storage 9. This communication may be performed in a wired or wireless manner.

The storage device 13 is, for example, a storage, and stores a program to be read and executed by the processor 16, a unique private key, and various data. The memory 14 temporarily holds data and programs. The memory 14 is a volatile memory, and is, for example, a random access memory (RAM). The output interface 15 can be connected to an external device and can output a signal to the external device. The processor 16 loads a program from the storage device 13 into the memory 14 and executes a series of instructions included in the program to execute various types of processing.

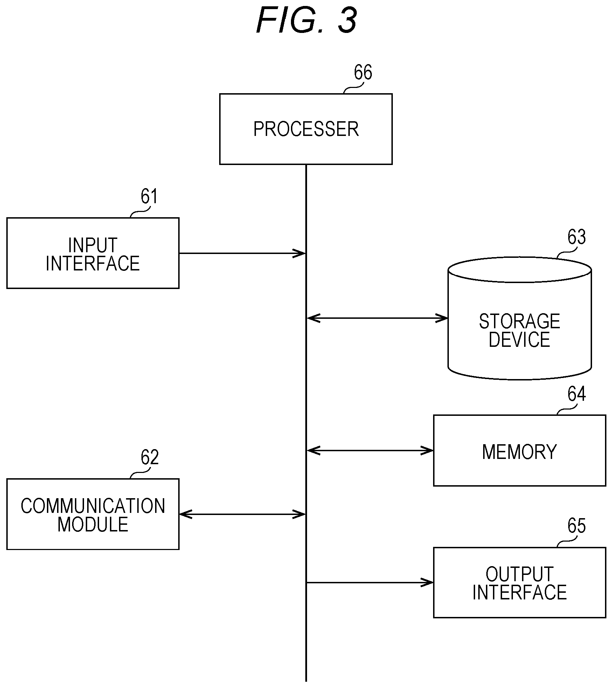

Fig. 3 is a schematic configuration diagram of the management system according to the present embodiment. As illustrated in Fig. 3, the management system 6 includes an input interface 61, a communication module 62, a storage device 63, a memory 64, an output interface 65, and a processor 66. Note that, here, as one aspect, the management system 6 will be described as including one processor 66, but there may be a plurality of processors, that is, one or more processors may be included. Furthermore, here, as one aspect, the management system 6 will be described as including one storage device 63, but there may be a plurality of storage devices, that is, one or more storage devices may be included.

The input interface 61 receives an input from an administrator (for example, an employee of the management organization) of the management system 6, and outputs an input signal corresponding to the received input to the processor 66.

The communication module 62 is connected to the communication network CN, and communicates with each of the terminals 1 to 5, the authentication system 7, the blockchain 81, and the storage 9. This communication may be performed in a wired or wireless manner.

The storage device 63 is, for example, a storage, and stores programs to be read and executed by the processor 66 and various data. The memory 64 temporarily holds data and programs. The memory 64 is a volatile memory, and is, for example, a random access memory (RAM). The output interface 65 can be connected to an external device and can output a signal to the external device. The processor 66 loads a program from the storage device 63 into the memory 64 and executes a series of instructions included in the program to execute various types of processing.

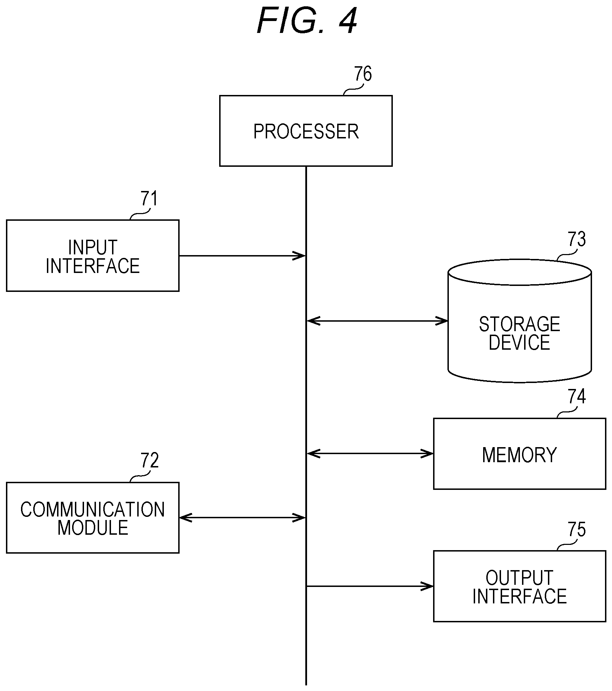

Fig. 4 is a schematic configuration diagram of the authentication system of the present embodiment. As illustrated in Fig. 4, the authentication system 7 includes an input interface 71, a communication module 72, a storage device 73, a memory 74, an output interface 75, and a processor 76. Note that, here, as one aspect, the authentication system 7 will be described as including one processor 76, but there may be a plurality of processors, that is, one or more processors may be included. Furthermore, here, as one aspect, the authentication system 7 will be described as including one storage device 73, but there may be a plurality of storage devices, that is, one or more storage devices may be included.

The input interface 71 receives an input from an administrator (for example, an employee of the authentication organization) of the authentication system 7, and outputs an input signal corresponding to the received input to the processor 76.

The communication module 72 is connected to the communication network CN and communicates with the management system 6 and the blockchain 81. This communication may be performed in a wired or wireless manner.

The storage device 73 is, for example, a storage, and stores a program to be read and executed by the processor 76, a public key (that is, five public keys) corresponding to a private key of each of the terminals 1 to 5, and various data. The memory 74 temporarily holds data and programs. The memory 74 is a volatile memory, and is, for example, a random access memory (RAM). The output interface 75 can be connected to an external device and can output a signal to the external device. The processor 76 loads a program from the storage device 73 into the memory 74 and executes a series of instructions included in the program to execute various types of processing.

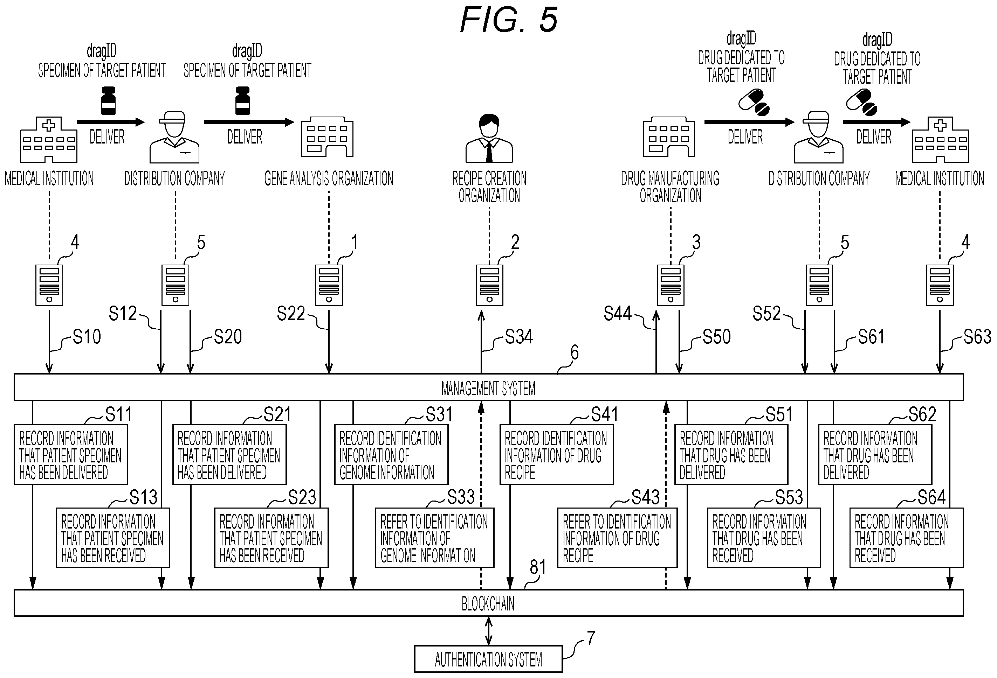

Fig. 5 is a schematic diagram illustrating an example of an outline of processing of the personalized drug creating process. In one aspect, first, in response to a request from the terminal 4 used by the medical institution, a dragID, which is an example of drug identification information for identifying a personalized drug, is issued from the management system 6.

First, a dragID and a specimen of a target patient (also referred to as a patient sample) are delivered from a medical institution to a distribution company. At that time, for example, the following processing is executed.

(Step S10) The terminal 4 transmits information indicating that the specimen of the target patient has been transferred to the management system 6.

(Step S11) The management system 6 records, in the blockchain 81, the information received from the terminal 4 indicating that the specimen of the target patient has been transferred.

(Step S12) The terminal 5 transmits information indicating that the specimen of the target patient has been received to the management system 6.

(Step S13) The management system 6 records, in the blockchain 81, the information received from the terminal 5 indicating that the specimen of the target patient has been received.

Next, the dragID and the specimen of the target patient are delivered from the distribution company to the gene analysis organization. At that time, for example, the following processing is executed.

(Step S20) The terminal 5 transmits information indicating that the specimen of the target patient has been delivered to the management system 6.

(Step S21) The management system 6 records, in the blockchain 81, the information received from the terminal 5 that the specimen of the target patient has been delivered.

(Step S22) The terminal 1 transmits information indicating that the specimen of the target patient has been received to the management system 6.

(Step S23) The management system 6 records, in the blockchain 81, the information received from the terminal 1 indicating that the specimen of the target patient has been received.

Next, genome information of the target patient is generated from the specimen of the genome target patient in the gene analysis organization. In one aspect, since the genome information has a large capacity, the identification information of the genome information is stored in the blockchain 81, and the genome information is stored in the storage 9. At that time, for example, the following processing is executed.

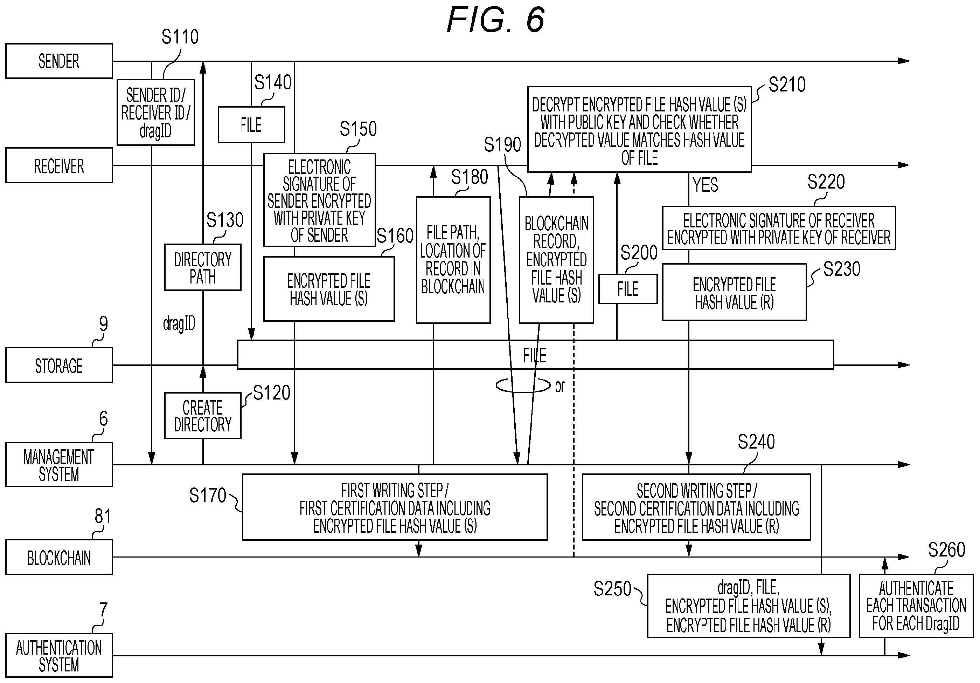

(Step S31) The management system 6 acquires the identification information of the genome information from the terminal 1, and records the identification information of the genome information in the blockchain 81. Here, the identification information of the genome information is, for example, a hash value of a file of the genome information encrypted by the terminal 1 using a private key of the terminal 1 (or a gene analysis organization) in step S160 of Fig. 6 to be described below. Note that the management system 6 may generate the identification information of the genome information. In the storage 9, for example, genome information is stored by the terminal 1 or the management system 6.

Next, the recipe creation organization creates a recipe for the personalized drug (for example, vaccines, specifically, for example, cancer vaccines). In one aspect, the genome information is referred to from the identification information of the genome information, and a recipe of the personalized drug is created. The recipe data of the personalized drug is recorded in the storage 9, and the identification information of the genome information is recorded in the blockchain 81. At that time, for example, the following processing is executed.

(Step S33) The management system 6 acquires the identification information of the genome information by referring to the blockchain 81 as an example. Here, the identification information of the genome information is, for example, a hash value of a file of the genome information encrypted by the terminal 1 using a private key of the terminal 1 (or a gene analysis organization) in step S160 of Fig. 6 to be described below.

(Step S34) Then, the management system 6 transmits the acquired identification information of the genome information to the terminal 2. Thereafter, steps S210 to S260 in Fig. 6 described below are executed.

(Step S41) Next, the management system 6 acquires recipe identification information of the personalized drug (for example, vaccines, specifically, for example, cancer vaccines) from the terminal 2, and records the recipe identification information in the blockchain. Here, the recipe identification information is, for example, a hash value of a file of recipe data encrypted by the terminal 2 using a private key of the terminal 2 (or a recipe creation organization) in step S160 of Fig. 6 to be described below. Note that the management system 6 may generate the recipe identification information. The recipe data of the personalized drug is recorded in the storage 9 by the terminal 2 or the management system 6.

Next, a personalized drug (for example, vaccines, specifically, for example, cancer vaccines) is manufactured in a drug manufacturing organization. From the recipe identification information of the personalized drug, recipe data of the personalized drug is referred to, and the drug manufacturing organization manufactures the personalized drug. At that time, for example, the following processing is executed.

(Step S43) The management system 6 acquires the recipe identification information with reference to the blockchain 81 as an example. Here, the recipe identification information is, for example, a hash value of a file of recipe data encrypted by the terminal 2 using a private key of the terminal 2 (or a recipe creation organization) in step S160 of Fig. 6 to be described below.

(Step S44) Then, the management system 6 transmits the acquired recipe identification information to the terminal 3. Thereafter, steps S210 to S260 in Fig. 6 described below are executed. The terminal 3 refers to the recipe data of the personalized drug from the recipe identification information and acquires the recipe data from the storage 9. The drug manufacturing organization manufactures the personalized drug from the recipe data of the personalized drug.

When the drug manufacturing organization delivers the manufactured personalized drug to the distribution company, for example, the following processing is executed.

(Step S50) The terminal 3 transmits the information about the drug delivery to the management system 6.

(Step S51) The management system 6 records, in the blockchain 81, the information received from the terminal 3 indicating that the drug has been delivered.

Next, when the distribution company receives the personalized drug from the drug manufacturing organization, for example, the following processing is executed.

(Step S52) The terminal 5 transmits information indicating that a drug has been received to the management system 6.

(Step S53) The management system 6 records, in the blockchain 81, the information received from the terminal 5 indicating that the drug has been received.

Next, in a case where the distribution company delivers the personalized drug to the medical institution, for example, the following processing is executed.

(Step S61) The terminal 5 transmits the information about the drug delivery to the management system 6.

(Step S62) The management system 6 records, in the blockchain 81, the information received from the terminal 5 indicating that the drug has been delivered.

Next, in a case where the medical institution receives the personalized drug from the distribution company, for example, the following processing is executed.

(Step S63) The terminal 4 transmits information indicating that a drug has been received to the management system 6.

(Step S64) The management system 6 records, in the blockchain 81, the information received from the terminal 4 indicating that the drug has been received.

As described above, the information on the delivering of the specimen of the target patient or the personalized drug is recorded in the blockchain 81, so that the delivering history is recorded without being erased or tampered. Therefore, it is possible to confirm the correct history during or after the personalized drug creating process.

Furthermore, as the genome identification information or the recipe identification information is recorded in the blockchain 81, the genome identification information or the recipe identification information is recorded without being erased or tampered. As a result, it is possible to acquire correct genome identification information or recipe identification information during or after the personalized drug creating process, so that it is possible to refer to correct genome information or recipe data and verify the correct genome information or recipe data.

Fig. 6 is a schematic diagram illustrating an example of a flow of transferring of the file of the target content. This processing is executed in each process of transferring the above target content. As an example, the description will be given assuming that the target content is genome information of the target patient, the sender is the terminal 1 used by the gene analysis organization, and the receiver is the terminal 2 used by the recipe creation organization.

(Step S110) The terminal 1 transmits the sender ID, the receiver ID, and the dragID that is identification information of the drug to the management system 6. In this example, the sender ID is information for identifying a gene analysis organization that uses the terminal 1, and the receiver ID is information for identifying a recipe creation organization that uses the terminal 2.

(Step S120) Next, the management system 6 creates a directory for target content (here, genome information as an example) in the storage 9.

(Step S130) Next, the management system 6 transmits a path of the created directory (also referred to as a directory path) to the terminal 1.

(Step S140) The terminal 1 that has received the directory path saves the file including the genome information in the directory indicated by the directory path of the storage 9.

(Step S150) Next, the terminal 1 transmits the electronic signature of the sender encrypted with the private key of the sender (here, the terminal 1 or the gene analysis organization as an example) to the management system 6.

(Step S160) Along with or in parallel with this as an example, the terminal 1 transmits the hash value of the file encrypted by the private key of the sender (here, the terminal 1 or the gene analysis organization as an example) to the management system 6. Here, the hash value of the file is a hash value of the file of the target content (here, the genome information as an example). In addition, the hash value of the file encrypted with the private key of the sender (here, as an example, the terminal 1 or the gene analysis organization) is also referred to as an encrypted file hash value (S) with "(S)" at the end indicating that the file is encrypted with the private key of the sender.

(Step S170) <First write step> Next, the management system 6 records the first verification data including the encrypted file hash value (S) in one block of the blockchain 81. This process is a specific example of step S31 in Fig. 5.

An example of the first verification data written to the blockchain in the first write step to the blockchain (step S180 in Fig. 6) is as follows.
dragID: drug identification information
encryptedContentHashValue_S: a hash value of the target content (here, the genome information as an example) encrypted with the private key of the sender
senderID: identification information of the sender (identification information of the creator of the hash value)
senderEsignature: an electronic signature of the sender (here, a gene analysis organization as an example)
recieverID: identification information of the receiver (here, the terminal 2 or the recipe creation organization as an example)
timestamp: time of delivery
location: delivery location

Note that the senderEsignature is the electronic signature of the sender, but is not limited thereto, and the electronic signature of the sender is encrypted with the private key of the sender, but is not limited thereto, and may be the electronic signature of the sender.

(Step S180) Next, the management system 6 transmits the file path and the position of the record of the blockchain recorded in step S170 to the terminal 2 that is the receiver.

(Step S190) For example, the terminal 2 that is the receiver requests the encrypted file hash value (S), and the management system 6 transmits the encrypted file hash value (S) and the blockchain record to the terminal 2 in response to the request.

Instead of the above processing in step S190, the management system 6 may transmit the encrypted file hash value (S) and the blockchain record to the terminal 2 without a request from the terminal 2.

(Step S200) In addition, the terminal 2 acquires a file including the target content (here, the genome information as an example) from the storage 9.

(Step S210) Next, the terminal 2 decrypts the encrypted file hash value (S) with the public key, collates the decrypted value with the hash value of the file acquired in step S200, and determines whether or not the decrypted value matches the hash value of the file. Here, the public key corresponds to the private key of the sender (here, the terminal 1 or the gene analysis organization as an example) used in the encryption in step S150. If decryption can be performed with the public key, it is proved that the encrypted file hash value (S) has been created by the owner of the private key (here, the terminal 1 or the gene analysis organization as an example). Further, when the decoded value matches the hash value of the file acquired in step S200, it is proved that the file acquired in step S200 is the true target content (here, the genome information as an example).

(Step S220) In a case where, in step S210, the value after decryption matches the hash value of the file acquired in step S200, the terminal 2 transmits the electronic signature of the receiver encrypted by the private key of the receiver (here, a recipe creation organization as an example) to the management system 6.

(Step S230) Along with or in parallel with this as an example, the terminal 2 transmits the hash value of the file encrypted by the private key of the receiver (here, the terminal 2 or the recipe creation organization as an example) to the management system 6. Here, the hash value of the file is a hash value of the file of the target content (here, the genome information as an example). In addition, the hash value of the file encrypted with the private key of the receiver (here, as an example, the terminal 2 or the recipe creation organization) is also referred to as an encrypted file hash value (R) with "(R)" added to the end indicating that the file is encrypted with the private key of the receiver.

(Step S240) <Second write step> Next, the management system 6 records the second verification data including the encrypted file hash value (R) in one block of the blockchain 81. The block recorded here is, for example, a block different from the block in step S170.

An example of the second verification data written to the blockchain in the second write step (step S240 in Fig. 6) of the blockchain is as follows.
dragID: drug identification information
encryptedContentHashValue_R: a hash value of the target content (here, the genome information as an example) encrypted with the private key of the receiver
recieverEsignature: an electronic signature of the receiver (here, as an example, a recipe creation organization)
timestamp: time of electronic signature

Note that the recieverEsignature is the electronic signature of the receiver, but the present invention is not limited thereto, and the electronic signature of the receiver may be encrypted with the private key of the receiver.

(Step S250) The management system 6 transmits the target dragID, the file of the target content (in this case, the genome information as an example), the encrypted file hash value (S), and the encrypted file hash value (R) to the authentication system 7.

(Step S260) When receiving the target dragID, the file of the target content (in this case, the genome information as an example), the encrypted file hash value (S), and the encrypted file hash value (R), the authentication system 7 verifies whether the transfer of the target content is appropriately performed in each exchange unit, and authenticates the fact that the transfer is appropriately performed as a result of the verification. Specifically, for example, the authentication system 7 executes the following processing. For example, the authentication system 7 searches the blockchain 81 for a block in the blockchain 81 using a dragID that is target drug identification information as a key, and authenticates that the target content is normally transferred when hash values included in the obtained two blocks match as a result of the search. Specifically, for example, the authentication system 7 acquires the file hash value (S) by decrypting the encrypted file hash value (S) included in one block with the public key corresponding to the private key of the terminal 1 that is the sender, acquires the file hash value (R) by decrypting the encrypted file hash value (R) with the public key corresponding to the private key of the terminal 2 that is the receiver, and determines whether or not the file hash value (S) and the file hash value (R) match.

As a result, it is possible to verify that the transfer of the target content (here, as an example, the genome information) has been appropriately performed and, if appropriate, to authenticate the fact. Note that the first verification data includes the first hash value encrypted with the first private key as an example of information based on the first hash value, but the present invention is not limited thereto, and the first hash value itself may be included.

To summarize the above, the information processing system 10 is an information processing system including a terminal 1, a terminal 2, and a management system 6.

The terminal 1 outputs a first hash value from data including at least genome information of the target patient, and transmits first verification data including the first hash value or information based on the first hash value (for example, a first hash value encrypted with a first private key). Here, the data including at least the genome information of the target patient may include at least one of the type of disease, medical history, HLA type, and sex of the target patient. The management system 6 records the first verification data received from the terminal 1 in the blockchain. The management system 6 may transmit storage location data (for example, a file path) indicating a storage location of the genome information to the terminal 2. The management system 6 reads the first verification data recorded in the blockchain and transmits the first verification data to the terminal 2. The terminal 2 acquires a first hash value from the first verification data received from the management system 6, reads the genome information from, for example, a storage area indicated by the received storage location data, generates a second hash value from the genome information, and determines whether or not the first hash value and the second hash value match.

With this configuration, when the first hash value recorded in the blockchain matches the second hash value generated from the genome information, it is ensured that both are the same genome information, and thus the authenticity of the genome information of the target patient can be ensured.

When transmitting the first verification data, the terminal 1 transmits the first hash value encrypted with a first private key. When acquiring the first hash value included in the first verification data, the terminal 2 acquires the first hash value by decrypting the first verification data with a first public key corresponding to the first private key. Note that the first public key may be held by the terminal 2 or may be input to the terminal 2 by a recipe creation organization using the terminal 2.

With this configuration, since only the gene analysis organization using the terminal 1 has the first private key, it is possible to ensure that the gene analysis organization has been involved in the encryption, and it is possible to ensure that the gene analysis organization has been involved in the generation of the genome information as a party, that is, to ensure the authenticity of the genome information creation party.

In addition, the first verification data received from the terminal 1 recorded in the blockchain 81 includes drug identification information for identifying the personalized drug for the target patient. When the first hash value matches the second hash value, the terminal 2 transmits second verification data including the drug identification information and the second hash value (specifically, for example, the file hash value or the encrypted file hash value (R)). In this case, since the second hash value matches the first hash value, the first hash value may be transmitted instead of the second hash value.
Subsequently, the management system 6 records the second verification data received from the terminal 2 in the blockchain.

With this configuration, in addition to the first verification data, the second verification data is further recorded in the blockchain. Thereafter, whether the transfer transaction of the genome information is normally performed can be verified by verifying whether the first hash value included in the first verification data recorded in the blockchain matches the second hash value included in the second verification data. This verification may be performed by the authentication system 7 as an example.

Specifically, for example, as described above, the authentication system 7 may search for a block in the blockchain using the target drug identification information as a key, and determine that the transfer of the genome information has been normally performed when the hash values included in the two obtained blocks match as a result of the search. With this configuration, it is possible to authenticate that the transfer of the genome information has been normally performed.

Note that, in Fig. 6, as an example, it has been described that the target content is genome information of the target patient, the sender is the terminal 1 used by the gene analysis organization, and the receiver is the terminal 2 used by the recipe creation organization, but the present invention is not limited thereto. The target content may be a vaccine recipe for the target patient, the sender may be the terminal 2 used by the recipe creation organization, and the receiver may be the terminal 3 used by the drug manufacturing organization.

In that case, a series of processes in Fig. 6 may be as follows.

(Step S110) The terminal 2 transmits the sender ID, the receiver ID, and the dragID as identification information of the drug to the management system 6. In this example, the sender ID is information for identifying a recipe creation organization using the terminal 2, and the receiver ID is information for identifying a drug manufacturing organization using the terminal 3.

(Step S120) Next, the management system 6 creates a directory for the target content (here, recipe data as an example) in the storage 9.

(Step S130) Next, the management system 6 transmits a path of the created directory (also referred to as a directory path) to the terminal 3.

(Step S140) When receiving the directory path, the terminal 3 saves the file including the recipe data in the directory indicated by the directory path of the storage 9.

(Step S150) Next, the terminal 2 transmits, to the management system 6, the electronic signature of the sender encrypted with the private key of the sender (here, the terminal 2 or the recipe creation organization as an example).

(Step S160) Along with or in parallel with this as an example, the terminal 2 transmits the hash value of the file encrypted by the private key of the sender (here, the terminal 2 or the recipe creation organization as an example) to the management system 6. Here, the hash value of the file is a hash value of the file of the target content (here, recipe data as an example). In addition, the hash value of the file encrypted by the private key of the sender (here, as an example, the terminal 2 or the recipe creation organization) is also referred to as an encrypted file hash value (S) with "(S)" at the end indicating that the file is encrypted by the private key of the sender.

(Step S170) <First write step> Next, the management system 6 records the first verification data including the encrypted file hash value (S) in one block of the blockchain 81. This process is a specific example of step S41 in Fig. 5.

An example of the first verification data written to the blockchain in the first write step to the blockchain (step S180 in Fig. 6) is as follows.
dragID: drug identification information
encryptedContentHashValue_S: a hash value of the target content (here, a file including the recipe data as an example) encrypted with the private key of the sender
senderID: identification information of the sender (identification information of the creator of the hash value)
senderEsignature: an electronic signature of the sender (here, as an example, a recipe creation organization)
recieverID: identification information of the receiver (here, the terminal 3 or the drug manufacturing organization as an example)
timestamp: time of delivery
location: delivery location

Note that the senderEsignature is the electronic signature of the sender, but is not limited thereto, and the electronic signature of the sender is encrypted with the private key of the sender, but is not limited thereto, and may be the electronic signature of the sender.

(Step S180) Next, the management system 6 transmits the file path and the position of the record of the blockchain recorded in step S170 to the terminal 3 that is the receiver.

(Step S190) For example, the terminal 3 that is the receiver requests the encrypted file hash value (S), and the management system 6 transmits the encrypted file hash value (S) and the blockchain record to the terminal 3 in response to the request.

In the process of step S190, instead of the above, the management system 6 may transmit the encrypted file hash value (S) and the blockchain record to the terminal 3 without a request from the terminal 3.

(Step S200) In addition, the terminal 2 acquires a file including the target content (here, recipe data as an example) from the storage 9.

(Step S210) Next, the terminal 2 decrypts the encrypted file hash value (S) with the public key, collates the decrypted value with the hash value of the file acquired in step S200, and determines whether or not the decrypted value matches the hash value of the file. Here, the public key corresponds to the private key of the sender (here, the terminal 2 or the recipe creation organization as an example) used in the encryption in step S150. If decryption can be performed with the public key, it is proved that the encrypted file hash value (S) has been created by the owner of the private key (here, the terminal 2 or the recipe creation organization as an example). Further, when the decoded value matches the hash value of the file acquired in step S200, it is proved that the file acquired in step S200 is the true target content (here, recipe data as an example).

(Step S220) In a case where, in step S210, the value after decryption matches the hash value of the file acquired in step S200, the terminal 3 transmits the electronic signature of the receiver encrypted by the private key of the receiver (here, as an example, a drug manufacturing organization) to the management system 6.

(Step S230) Along with or in parallel with this as an example, the terminal 3 transmits the hash value of the file encrypted with the private key of the receiver (here, the terminal 3 or the drug manufacturing organization as an example) to the management system 6. Here, the hash value of the file is a hash value of the file of the target content (here, recipe data as an example). In addition, the hash value of the file encrypted with the private key of the receiver (here, as an example, the terminal 3 or the drug manufacturing organization) is also referred to as an encrypted file hash value (R) with "(R)" added to the end indicating that the file is encrypted with the private key of the receiver.

(Step S240) <Second write step> Next, the management system 6 records the second verification data including the encrypted file hash value (R) in one block of the blockchain 81. The block recorded here is, for example, a block different from the block in step S170.

An example of the second verification data written to the blockchain in the second write step (step S240 in Fig. 6) of the blockchain is as follows.
dragID: drug identification information
encryptedContentHashValue_R: a hash value of the target content (here, recipe data as an example) encrypted with the private key of the receiver
recieverEsignature: an electronic signature of the receiver (here, as an example, a drug manufacturing organization).
timestamp: time of electronic signature

Note that the recieverEsignature is the electronic signature of the receiver, but the present invention is not limited thereto, and the electronic signature of the receiver may be encrypted with the private key of the receiver.

(Step S250) The management system 6 transmits the target dragID, the file of the target content (here, as an example, recipe data), the encrypted file hash value (S), and the encrypted file hash value (R) to the authentication system 7.

(Step S260) When receiving the target dragID, the file of the target content (here, as an example, the recipe data), the encrypted file hash value (S), and the encrypted file hash value (R), the authentication system 7 verifies whether the transfer of the target content is appropriately performed in each exchange unit, and authenticates the fact that the transfer is appropriately performed as a result of the verification. Specifically, for example, the authentication system 7 executes the following processing. For example, the authentication system 7 searches the blockchain 81 for a block in the blockchain 81 using a dragID that is target drug identification information as a key, and authenticates that the target content is normally transferred when hash values included in the obtained two blocks match as a result of the search. Specifically, for example, the authentication system 7 acquires the file hash value (S) by decrypting the encrypted file hash value (S) included in one block with the public key corresponding to the private key of the terminal 2 that is the sender, acquires the file hash value (R) by decrypting the encrypted file hash value (R) with the public key corresponding to the private key of the terminal 3 that is the receiver, and determines whether or not the file hash value (S) and the file hash value (R) match.

As a result, it is possible to verify that the transfer of the target content (here, as an example, the recipe data) has been appropriately performed and, if appropriate, to authenticate the fact. Note that the third verification data includes the third hash value encrypted with the third private key as an example of the information based on the third hash value, but the present invention is not limited thereto, and the third hash value itself may be included.

In this manner, the terminal 2 outputs the third hash value from the data including at least the recipe of the drug for the target patient (for example, the vaccine recipe), and transmits the third verification data including the third hash value or the information based on the third hash value (for example, the third hash value encrypted with the third private key). The management system 6 records the third verification data received from the terminal 2 in the blockchain. The management system 6 may transmit storage location data (for example, a file path) indicating a storage location of the recipe to the terminal 2. The management system 6 transmits the third verification data recorded in the blockchain to the terminal 3. The terminal 3 acquires a third hash value from the third verification data received from the management system 6, reads the recipe from the storage area indicated by the received storage location data, generates a fourth hash value from the recipe, and determines whether or not the third hash value matches the fourth hash value.

With this configuration, when the first hash value recorded in the blockchain and the fourth hash value generated from the recipe match, it is ensured that both are the same recipe, so that the authenticity of the recipe can be ensured.

When transmitting the third verification data, the terminal 2 may transmit a result of encrypting the third hash value with a second private key. In this case, when acquiring the third hash value included in the third verification data, the terminal 3 may acquire the second hash value by decrypting the third verification data with a second public key corresponding to the second private key.

With this configuration, since only the recipe creation organization using the terminal 2 has the second private key, it is possible to ensure that the recipe creation organization has been involved in the encryption, and it is possible to ensure that the recipe creation organization has been involved in the generation of the recipe as a party, that is, to ensure the authenticity of the recipe creation party.

In addition, the third verification data received from the terminal 2 recorded in the blockchain 81 may include drug identification information for identifying the personalized drug for the target patient. In this case, when the third hash value matches the fourth hash value, the terminal 3 may transmit fourth verification data including the drug identification information and the third hash value or the fourth hash value. In this case, the management system 6 may record the fourth verification data received from the terminal 3 in the blockchain.

With this configuration, in addition to the third verification data, the fourth verification data is further recorded in the blockchain. Thereafter, by verifying whether the third hash value included in the third verification data recorded in the blockchain matches the fourth hash value included in the fourth verification data, it is possible to verify whether the transfer transaction of the recipe has been normally performed. This verification is executed by the authentication system 7 as an example.

Specifically, for example, the authentication system 7 may search for a block in the blockchain using the target drug identification information as a key, and determine that the transfer of the recipe has been normally performed when the hash values included in the obtained two blocks match as a result of the search. With this configuration, it is possible to authenticate that the transfer of the recipe has been normally performed.

In addition, the terminal 4 may transmit fifth verification data for proving that the specimen of the target patient is delivered from the medical institution to the distribution company. In this case, the management system 6 may record the fifth verification data received from the terminal 4 in the blockchain 81. Furthermore, in this case, the terminal 5 may transmit sixth verification data for proving that the distribution company has received the specimen of the target patient from the medical institution. Furthermore, in this case, the management system 6 may record the sixth verification data received from the terminal 5 in the blockchain 81.

With this configuration, it can be verified later that the specimen of the target patient has been normally delivered.

Here, the fifth verification data may include a hash value of the document data of the personalized drug encrypted with a third private key for a medical institution. The sixth verification data may include a hash value of the document data of the personalized drug encrypted with a fourth private key for a distribution company.

As described above, the information processing system 10 according to the present embodiment is an information processing system including a first terminal, a second terminal, and a management system, in which the first terminal outputs a first hash value from data including at least target content, and transmits first verification data including the first hash value or information based on the first hash value (for example, a first hash value encrypted with a first private key). The management system records the first verification data received from the first terminal in the blockchain. The management system transmits the first verification data recorded in the blockchain to the second terminal. The second terminal acquires the first hash value from the first verification data received from the management system. In a case where the information based on the first hash value included in the first verification data is obtained by encrypting the first hash value with the first private key, decryption with the first public key corresponding to the first private key is included during the acquisition process. Then, for example, the second terminal acquires the target content from a storage area of a storage indicated by storage location data (for example, a file path) received from the management system, generates a second hash value from the target content, and determines whether or not the first hash value matches the second hash value.

Here, the first terminal and the second terminal may be the terminal 1 and the terminal 2, may be the terminal 2 and the terminal 3, may be the terminal 2 and the terminal 3, may be the terminal 4 and the terminal 5, may be the terminal 5 and the terminal 1, may be the terminal 3 and the terminal 4, or may be the terminal 5 and the terminal 4, respectively.

As a result, when the first hash value recorded in the blockchain matches the second hash value generated from the target content, it is ensured that the first hash value and the second hash value are the same target content. Therefore, authenticity of the target content can be ensured.

Although the storage 9 has been described as a separate body from the management system 6, the present invention is not limited thereto, and the storage 9 may be built in the management system 6. Note that, in one aspect described above, as an example, target content (for example, genome information or recipe data) is exchanged via the storage 9. In a case where the target content has a large amount of data such as genome information, for example, the target content can be smoothly exchanged via the storage 9. However, the present invention is not limited to thereto, and the target content may be exchanged without the storage 9. For example, the second terminal may directly receive the target content from the first terminal or the management system, or a user who uses the second terminal may physically receive the memory storing the target content by delivery or the like and store the memory in the second terminal. As long as the second terminal can acquire the target content, the acquisition route is not limited.

Note that at least a part of the management system 6 or the terminals 1 to 5 described in the above-described embodiment may be configured by hardware or software. In the case of being configured by software, a program for realizing at least some functions of the management system 6 or the terminals 1 to 5 may be stored in a computer-readable recording medium and read and executed by a computer. The recording medium is not limited to a removable recording medium such as a magnetic disk or an optical disk, and may be a fixed recording medium such as a hard disk device or a memory.

In addition, a program for realizing at least some functions of the management system 6 or the terminals 1 to 5 may be distributed via a communication line (including wireless communication) such as the Internet. Further, the program may be distributed via a wired line or a wireless line such as the Internet or by being stored in a recording medium in an encrypted, modulated, or compressed state.

Furthermore, the management system 6 may be caused to function by one or a plurality of information devices. In the case of using a plurality of information devices, one of the information devices may be a computer, and the computer may execute a predetermined program to implement a function as at least one unit of the management system 6.

In the invention of the method, all the processes (steps) may be realized by automatic control by a computer. In addition, the progress control between the processes may be performed by a human hand while causing a computer to perform each process. Furthermore, at least a part of all the processes may be performed by a human hand.

As described above, the present invention is not limited to the above-described embodiment as it is, and can be embodied by modifying the components in the implementation stage. In addition, various inventions can be formed by appropriately combining a plurality of constituent elements disclosed in the above embodiment. For example, some components may be deleted from all the components shown in the embodiments. Furthermore, constituent elements in different embodiments may be appropriately combined. The invention is defined by the appended claims.

### Reference Signs List

- 1: Terminal
- 10: Information processing system
- 11: Input interface
- 12: Communication module
- 13: Storage device
- 14: Memory
- 15: Output interface
- 16: Processor
- 2: Terminal
- 3: Terminal
- 4: Terminal
- 5: Terminal
- 6: Management system
- 61: Input interface
- 62: Communication module
- 63: Storage device
- 64: Memory
- 65: Output interface
- 66: Processor
- 7: Authentication system
- 71: Input interface
- 72: Communication module
- 73: Storage device
- 74: Memory
- 75: Output interface
- 76: Processor
- 8: Distributed network
- 81: Blockchain
- 9: Storage

## Claims

1. An information processing system (10) comprising: a first terminal; a second terminal; a management system (6); and an authentication system (7), wherein
the first terminal outputs a first hash value from data including at least target content, and transmits first verification data including either the first hash value or information based on the first hash value,
the management system records the first verification data including drug identification information for identifying a personalized drug for a target patient received from the first terminal in a blockchain,
the management system transmits the first verification data recorded in the blockchain to the second terminal, and
the second terminal acquires a first hash value from the first verification data received from the management system, acquires the target content and generates a second hash value from the acquired target content, determines whether the first hash value matches the second hash value, and in a case where the first hash value matches the second hash value, the second terminal transmits second verification data including the drug identification information and the first hash value or the second hash value,
the management system records the second verification data received from the second terminal in the blockchain,
the authentication system searches for blocks in the blockchain using the drug identification information as a key, in a case where hash values included in two obtained blocks match as a result of the search, determines that the transfer of the target content has been performed normally, and otherwise, determines that the transfer of the target content has been performed abnormally.

2. The information processing system according to claim 1, wherein the first terminal is used by a gene analysis organization that generates genome information of a target patient from a specimen of the target patient,
the second terminal is used by a recipe creation organization that creates a vaccine recipe, and
the target content is genome information of the target patient.

3. The information processing system according to claim 2, wherein the genome information of the target patient includes gene data of cancer cells of the target patient and gene data of normal cells of the target patient.

4. The information processing system according to claim 1, wherein
the first terminal is used by a recipe creation organization that creates a vaccine recipe,
the second terminal is used by a drug manufacturing organization that manufactures a personalized drug from the vaccine recipe, and
the target content is a vaccine recipe for a target patient.

5. The information processing system according to any one of claims 1 to 4, wherein the information based on the first hash value is obtained by encrypting the first hash value with a first private key,
when transmitting the first verification data, the first terminal transmits the first hash value encrypted with the first private key, and
when acquiring the first hash value included in the first verification data, the second terminal acquires the first hash value by decrypting the first verification data with a first public key corresponding to the first private key.

6. The information processing system according to claim 2, further comprising a third terminal used by a drug manufacturing organization that manufactures a personalized drug from a vaccine recipe, wherein
the second terminal outputs a hash value from data including at least a recipe of a drug for a target patient, and transmits third verification data including either the hash value or information based on the hash value,
the management system records the third verification data received from the second terminal in a blockchain,
the management system transmits the third verification data recorded in the blockchain to the third terminal, and
the third terminal acquires a fourth hash value included in the third verification data received from the management system, acquires the recipe and generates a fourth hash value from the acquired recipe, and determines whether the third hash value matches the fourth hash value.

7. The information processing system according to claim 6, wherein when transmitting the third verification data, the second terminal transmits the third hash value encrypted with a second private key, and
when acquiring the third hash value included in the third verification data, the third terminal acquires the second hash value by decrypting the third verification data with a second public key corresponding to the second private key.

8. The information processing system according to claim 6 or 7, wherein the third verification data received from the second terminal recorded in the blockchain includes drug identification information for identifying a personalized drug for a target patient,
in a case where the third hash value matches the fourth hash value, the third terminal transmits fourth verification data including the drug identification information and the third hash value or the fourth hash value, and
the management system records the fourth verification data received from the third terminal in the blockchain.

9. The information processing system according to any one of claims 1 to 8, further comprising: a fourth terminal used by a medical institution; and a fifth terminal used by a distribution company, wherein
the fourth terminal transmits fifth verification data for proving that a specimen of a target patient has been delivered from the medical institution to the distribution company,
the management system records the fifth verification data received from the fourth terminal in a blockchain,
the fifth terminal transmits sixth verification data for proving that the distribution company has received a specimen of a target patient from the medical institution, and
the management system records the sixth verification data received from the fifth terminal in a blockchain.

10. The information processing system according to claim 9, wherein the fifth verification data includes a hash value of document data of the personalized drug encrypted with a third private key for a medical institution, and
the sixth verification data includes a hash value of the document data of the personalized drug encrypted with a fourth private key for a distribution company.

11. An information processing method comprising: outputting, by a first terminal, a first hash value from data including at least target content, and transmitting first verification data including either the first hash value or information based on the first hash value;
recording, by a management system, the first verification data including drug identification information for identifying a personalized drug for a target patient received from the first terminal in a blockchain;
transmitting, by the management system, the first verification data recorded in the blockchain to a second terminal; and
acquiring, by the second terminal, a first hash value from the first verification data received from the management system, acquiring the target content and generating a second hash value from the acquired target content,
determining whether the first hash value matches the second hash value, wherein, in a case where the first hash value matches the second hash value, the second terminal transmits second verification data including the drug identification information and the first hash value or the second hash value,
the management system records the second verification data received from the second terminal in the blockchain, and
an authentication system searches for blocks in the blockchain using the drug identification information as a key, in a case where hash values included in two obtained blocks match as a result of the search, determines that the transfer of the target content has been performed normally, and otherwise, determines that the transfer of the target content has been performed abnormally.

12. A program for causing a plurality of processors to execute a method according to claim 11.

## Patentansprüche

1. Informationsverarbeitungssystem (10), umfassend: ein erstes Endgerät; ein zweites Endgerät; ein Verwaltungssystem (6); und ein Authentifizierungssystem (7), wobei
das erste Endgerät einen ersten Hash-Wert aus Daten ausgibt, die zumindest Zielinhalt enthalten, und erste Verifizierungsdaten überträgt, die entweder den ersten Hash-Wert oder Informationen basierend auf dem ersten Hash-Wert enthalten,
das Verwaltungssystem die ersten Verifizierungsdaten, die von dem ersten Endgerät empfangen wurden und Arzneimittelidentifikationsinformationen zum Identifizieren eines personalisierten Arzneimittels für einen Zielpatienten enthalten, in einer Blockchain aufzeichnet,
das Verwaltungssystem die in der Blockchain aufgezeichneten ersten Verifizierungsdaten an das zweite Endgerät überträgt, und
das zweite Endgerät einen ersten Hash-Wert aus den von dem Verwaltungssystem empfangenen ersten Verifizierungsdaten erfasst, den Zielinhalt erfasst und einen zweiten Hash-Wert aus dem erfassten Zielinhalt erzeugt, bestimmt, ob der erste Hash-Wert mit dem zweiten Hash-Wert übereinstimmt, und in einem Fall, in dem der erste Hash-Wert mit dem zweiten Hash-Wert übereinstimmt, das zweite Endgerät zweite Verifizierungsdaten überträgt, die die Arzneimittelidentifikationsinformationen und den ersten Hash-Wert oder den zweiten Hash-Wert enthalten,
das Verwaltungssystem die von dem zweiten Endgerät empfangenen zweiten Verifizierungsdaten in der Blockchain aufzeichnet,
das Authentifizierungssystem nach Blöcken in der Blockchain unter Verwendung der Arzneimittelidentifikationsinformationen als Schlüssel sucht, in einem Fall, in dem in zwei erhaltenen Blöcken enthaltene Hash-Werte als ein Ergebnis der Suche übereinstimmen, bestimmt, dass die Übertragung des Zielinhalts normal durchgeführt wurde, und andernfalls bestimmt, dass die Übertragung des Zielinhalts unnormal durchgeführt wurde.

2. Informationsverarbeitungssystem nach Anspruch 1, wobei das erste Endgerät von einer Genanalyseorganisation verwendet wird, die Genominformationen eines Zielpatienten aus einer Probe des Zielpatienten erzeugt,
das zweite Endgerät von einer Rezepturerstellungsorganisation verwendet wird, die eine Impfstoffrezeptur erstellt, und
der Zielinhalt Genominformationen des Zielpatienten sind.

3. Informationsverarbeitungssystem nach Anspruch 2, wobei die Genominformationen des Zielpatienten Gendaten von Krebszellen des Zielpatienten und Gendaten von normalen Zellen des Zielpatienten enthalten.

4. Informationsverarbeitungssystem nach Anspruch 1, wobei
das erste Endgerät von einer Rezepturerstellungsorganisation verwendet wird, die eine Impfstoffrezeptur erstellt,
das zweite Endgerät von einer Arzneimittelherstellungsorganisation verwendet wird, die ein personalisiertes Arzneimittel aus der Impfstoffrezeptur herstellt, und
der Zielinhalt eine Impfstoffrezeptur für einen Zielpatienten ist.

5. Informationsverarbeitungssystem nach einem der Ansprüche 1 bis 4, wobei die Informationen basierend auf dem ersten Hash-Wert durch Verschlüsseln des ersten Hash-Werts mit einem ersten privaten Schlüssel erhalten werden,
wenn die ersten Verifizierungsdaten übertragen werden, das erste Endgerät den mit dem ersten privaten Schlüssel verschlüsselten ersten Hash-Wert überträgt, und
wenn der in den ersten Verifizierungsdaten enthaltene erste Hash-Wert erfasst wird, das zweite Endgerät den ersten Hash-Wert durch Entschlüsseln der ersten Verifizierungsdaten mit einem ersten öffentlichen Schlüssel, der dem ersten privaten Schlüssel entspricht, erfasst.

6. Informationsverarbeitungssystem nach Anspruch 2, ferner umfassend ein drittes Endgerät, das von einer Arzneimittelherstellungsorganisation verwendet wird, die ein personalisiertes Arzneimittel aus einer Impfstoffrezeptur herstellt, wobei
das zweite Endgerät einen Hash-Wert aus Daten ausgibt, die zumindest eine Rezeptur eines Arzneimittels für einen Zielpatienten enthalten, und dritte Verifizierungsdaten überträgt, die entweder den Hash-Wert oder Informationen basierend auf dem Hash-Wert enthalten,
das Verwaltungssystem die von dem zweiten Endgerät empfangenen dritten Verifizierungsdaten in einer Blockchain aufzeichnet,
das Verwaltungssystem die in der Blockchain aufgezeichneten dritten Verifizierungsdaten an das dritte Endgerät überträgt, und
das dritte Endgerät einen vierten Hash-Wert erfasst, der in den von dem Verwaltungssystem empfangenen dritten Verifizierungsdaten enthalten ist, die Rezeptur erfasst und einen vierten Hash-Wert aus der erfassten Rezeptur erzeugt, und bestimmt, ob der dritte Hash-Wert mit dem vierten Hash-Wert übereinstimmt.

7. Informationsverarbeitungssystem nach Anspruch 6, wobei, wenn die dritten Verifizierungsdaten übertragen werden, das zweite Endgerät den mit einem zweiten privaten Schlüssel verschlüsselten dritten Hash-Wert überträgt, und
wenn der in den dritten Verifizierungsdaten enthaltene dritte Hash-Wert erfasst wird, das dritte Endgerät den zweiten Hash-Wert durch Entschlüsseln der dritten Verifizierungsdaten mit einem zweiten öffentlichen Schlüssel, der dem zweiten privaten Schlüssel entspricht, erfasst.

8. Informationsverarbeitungssystem nach Anspruch 6 oder 7, wobei die von dem zweiten Endgerät empfangenen dritten Verifizierungsdaten, die in der Blockchain aufgezeichnet sind, Arzneimittelidentifikationsinformationen zum Identifizieren eines personalisierten Arzneimittels für einen Zielpatienten enthalten,
in einem Fall, in dem der dritte Hash-Wert mit dem vierten Hash-Wert übereinstimmt, das dritte Endgerät vierte Verifizierungsdaten überträgt, die die Arzneimittelidentifikationsinformationen und den dritten Hash-Wert oder den vierten Hash-Wert enthalten, und
das Verwaltungssystem die von dem dritten Endgerät empfangenen vierten Verifizierungsdaten in der Blockchain aufzeichnet.

9. Informationsverarbeitungssystem nach einem der Ansprüche 1 bis 8, ferner umfassend:
ein viertes Endgerät, das von einer medizinischen Einrichtung verwendet wird; und ein fünftes Endgerät, das von einem Vertriebsunternehmen verwendet wird, wobei
das vierte Endgerät fünfte Verifizierungsdaten zum Nachweis überträgt, dass eine Probe eines Zielpatienten von der medizinischen Einrichtung an das Vertriebsunternehmen geliefert wurde,
das Verwaltungssystem die von dem vierten Endgerät empfangenen fünften Verifizierungsdaten in einer Blockchain aufzeichnet,
das fünfte Endgerät sechste Verifizierungsdaten zum Nachweis überträgt, dass das Vertriebsunternehmen eine Probe eines Zielpatienten von der medizinischen Einrichtung erhalten hat, und
das Verwaltungssystem die von dem fünften Endgerät empfangenen sechsten Verifizierungsdaten in einer Blockchain aufzeichnet.

10. Informationsverarbeitungssystem nach Anspruch 9, wobei die fünften Verifizierungsdaten einen Hash-Wert von Dokumentdaten des personalisierten Arzneimittels enthalten, der mit einem dritten privaten Schlüssel für eine medizinische Einrichtung verschlüsselt ist, und
die sechsten Verifizierungsdaten einen Hash-Wert der Dokumentdaten des personalisierten Arzneimittels enthalten, der mit einem vierten privaten Schlüssel für ein Vertriebsunternehmen verschlüsselt ist.

11. Informationsverarbeitungsverfahren, umfassend:
Ausgeben, durch ein erstes Endgerät, eines ersten Hash-Werts aus Daten, die zumindest Zielinhalt enthalten, und Übertragen erster Verifizierungsdaten, die entweder den ersten Hash-Wert oder Informationen basierend auf dem ersten Hash-Wert enthalten;
Aufzeichnen, durch ein Verwaltungssystem, der ersten Verifizierungsdaten, die von dem ersten Endgerät empfangen wurden und Arzneimittelidentifikationsinformationen zum Identifizieren eines personalisierten Arzneimittels für einen Zielpatienten enthalten, in einer Blockchain;
Übertragen, durch das Verwaltungssystem, der in der Blockchain aufgezeichneten ersten Verifizierungsdaten an ein zweites Endgerät; und
Erfassen, durch das zweite Endgerät, eines ersten Hash-Werts aus den von dem Verwaltungssystem empfangenen ersten Verifizierungsdaten, Erfassen des Zielinhalts und Erzeugen eines zweiten Hash-Werts aus dem erfassten Zielinhalt, Bestimmen, ob der erste Hash-Wert mit dem zweiten Hash-Wert übereinstimmt, wobei in einem Fall, in dem der erste Hash-Wert mit dem zweiten Hash-Wert übereinstimmt, das zweite Endgerät zweite Verifizierungsdaten überträgt, die die Arzneimittelidentifikationsinformationen und den ersten Hash-Wert oder den zweiten Hash-Wert enthalten,
das Verwaltungssystem zeichnet die von dem zweiten Endgerät empfangenen zweiten Verifizierungsdaten in der Blockchain auf, und
ein Authentifizierungssystem sucht nach Blöcken in der Blockchain unter Verwendung der Arzneimittelidentifikationsinformationen als Schlüssel, bestimmt in einem Fall, in dem in zwei erhaltenen Blöcken enthaltene Hash-Werte als ein Ergebnis der Suche übereinstimmen, dass die Übertragung des Zielinhalts normal durchgeführt wurde, und bestimmt andernfalls, dass die Übertragung des Zielinhalts unnormal durchgeführt wurde.

12. Programm, um eine Vielzahl von Prozessoren zu veranlassen, ein Verfahren nach Anspruch 11 auszuführen.

## Revendications

1. Système de traitement d'informations (10) comportant : un premier terminal ; un deuxième terminal ; un système de gestion (6) ; et un système d'authentification (7), dans lequel
le premier terminal délivre en sortie une première valeur de hachage à partir de données incluant au moins un contenu cible, et transmet de premières données de vérification incluant soit la première valeur de hachage, soit des informations basées sur la première valeur de hachage,
le système de gestion enregistre les premières données de vérification incluant des informations d'identification de médicament permettant d'identifier un médicament personnalisé pour un patient cible reçues du premier terminal dans une chaîne de blocs,
le système de gestion transmet les premières données de vérification enregistrées dans la chaîne de blocs au deuxième terminal, et
le deuxième terminal acquiert une première valeur de hachage à partir des premières données de vérification reçues du système de gestion, acquiert le contenu cible et génère une deuxième valeur de hachage à partir du contenu cible acquis, détermine si la première valeur de hachage correspond à la deuxième valeur de hachage, et dans un cas où la première valeur de hachage correspond à la deuxième valeur de hachage, le deuxième terminal transmet de deuxièmes données de vérification incluant les informations d'identification de médicament et la première valeur de hachage ou la deuxième valeur de hachage,
le système de gestion enregistre les deuxièmes données de vérification reçues du deuxième terminal dans la chaîne de blocs,
le système d'authentification recherche des blocs dans la chaîne de blocs en utilisant les informations d'identification de médicament comme clé, dans un cas où des valeurs de hachage incluses dans deux blocs obtenus correspondent à la suite de la recherche, détermine que le transfert du contenu cible a été réalisé normalement, et dans le cas contraire, détermine que le transfert du contenu cible a été réalisé anormalement.

2. Système de traitement d'informations selon la revendication 1, dans lequel le premier terminal est utilisé par une organisation d'analyse génétique qui génère des informations génomiques d'un patient cible à partir d'un échantillon du patient cible,
le deuxième terminal est utilisé par une organisation de création de recette qui crée une recette de vaccin, et
le contenu cible est les informations génomiques du patient cible.

3. Système de traitement d'informations selon la revendication 2, dans lequel les informations génomiques du patient cible incluent des données génétiques de cellules cancéreuses du patient cible et des données génétiques de cellules normales du patient cible.

4. Système de traitement d'informations selon la revendication 1, dans lequel
le premier terminal est utilisé par une organisation de création de recette qui crée une recette de vaccin,
le deuxième terminal est utilisé par une organisation de fabrication de médicament qui fabrique un médicament personnalisé à partir de la recette de vaccin, et
le contenu cible est une recette de vaccin pour un patient cible.

5. Système de traitement d'informations selon l'une quelconque des revendications 1 à 4, dans lequel les informations basées sur la première valeur de hachage sont obtenues en chiffrant la première valeur de hachage avec une première clé privée,
lors de la transmission des premières données de vérification, le premier terminal transmet la première valeur de hachage chiffrée avec la première clé privée, et
lors de l'acquisition de la première valeur de hachage incluse dans les premières données de vérification, le deuxième terminal acquiert la première valeur de hachage en déchiffrant les premières données de vérification avec une première clé publique correspondant à la première clé privée.

6. Système de traitement d'informations selon la revendication 2, comportant en outre un troisième terminal utilisé par une organisation de fabrication de médicament qui fabrique un médicament personnalisé à partir d'une recette de vaccin, dans lequel
le deuxième terminal délivre en sortie une valeur de hachage à partir de données incluant au moins une recette d'un médicament pour un patient cible, et transmet de troisièmes données de vérification incluant soit la valeur de hachage, soit des informations basées sur la valeur de hachage,
le système de gestion enregistre les troisièmes données de vérification reçues du deuxième terminal dans une chaîne de blocs,
le système de gestion transmet les troisièmes données de vérification enregistrées dans la chaîne de blocs au troisième terminal, et
le troisième terminal acquiert une quatrième valeur de hachage incluse dans les troisièmes données de vérification reçues du système de gestion, acquiert la recette et génère une quatrième valeur de hachage à partir de la recette acquise, puis détermine si la troisième valeur de hachage correspond à la quatrième valeur de hachage.

7. Système de traitement d'informations selon la revendication 6, dans lequel, lors de la transmission des troisièmes données de vérification, le deuxième terminal transmet la troisième valeur de hachage chiffrée avec une deuxième clé privée, et
lors de l'acquisition de la troisième valeur de hachage incluse dans les troisièmes données de vérification, le troisième terminal acquiert la deuxième valeur de hachage en déchiffrant les troisièmes données de vérification avec une deuxième clé publique correspondant à la deuxième clé privée.

8. Système de traitement d'informations selon la revendication 6 ou 7, dans lequel les troisièmes données de vérification reçues du deuxième terminal enregistrées dans la chaîne de blocs incluent des informations d'identification de médicament permettant d'identifier un médicament personnalisé pour un patient cible,
dans le cas où une troisième valeur de hachage correspond à la quatrième valeur de hachage, le troisième terminal transmet de quatrièmes données de vérification incluant les informations d'identification de médicament et la troisième valeur de hachage ou la quatrième valeur de hachage, et
le système de gestion enregistre les quatrièmes données de vérification reçues du troisième terminal dans la chaîne de blocs.

9. Système de traitement d'informations selon l'une quelconque des revendications 1 à 8, comportant en outre : un quatrième terminal utilisé par un établissement médical ; et un cinquième terminal utilisé par une société de distribution, dans lequel
le quatrième terminal transmet de cinquièmes données de vérification pour prouver qu'un échantillon d'un patient cible a été livré de l'établissement médical à la société de distribution,
le système de gestion enregistre les cinquièmes données de vérification reçues du quatrième terminal dans une chaîne de blocs,
le cinquième terminal transmet de sixièmes données de vérification pour prouver que la société de distribution a reçu un échantillon d'un patient cible de l'établissement médical, et
le système de gestion enregistre les sixièmes données de vérification reçues du cinquième terminal dans une chaîne de blocs.

10. Système de traitement d'informations selon la revendication 9, dans lequel les cinquièmes données de vérification incluent une valeur de hachage de données de document du médicament personnalisé chiffrée avec une troisième clé privée pour un établissement médical, et
les sixièmes données de vérification incluent une valeur de hachage des données de document du médicament personnalisé chiffrée avec une quatrième clé privée pour une société de distribution.

11. Procédé de traitement d'informations comportant :
la sortie, par un premier terminal, d'une première valeur de hachage à partir de données incluant au moins un contenu cible, et la transmission de premières données de vérification incluant soit la première valeur de hachage, soit des informations basées sur la première valeur de hachage ;
l'enregistrement, par un système de gestion, des premières données de vérification incluant des informations d'identification de médicament permettant d'identifier un médicament personnalisé pour un patient cible reçues du premier terminal dans une chaîne de blocs ;
la transmission, par le système de gestion, des premières données de vérification enregistrées dans la chaîne de blocs à un deuxième terminal ; et
l'acquisition, par le deuxième terminal, d'une première valeur de hachage à partir des premières données de vérification reçues du système de gestion, l'acquisition du contenu cible et la génération d'une deuxième valeur de hachage à partir du contenu cible acquis,
le fait de déterminer si la première valeur de hachage correspond à la deuxième valeur de hachage, dans un cas où la première valeur de hachage correspond à la deuxième valeur de hachage, le deuxième terminal transmet de deuxièmes données de vérification incluant les informations d'identification de médicament et la première valeur de hachage ou la deuxième valeur de hachage,
le système de gestion enregistre les deuxièmes données de vérification reçues du deuxième terminal dans la chaîne de blocs, et
un système d'authentification recherche des blocs dans la chaîne de blocs en utilisant les informations d'identification de médicament comme clé, dans un cas où des valeurs de hachage incluses dans deux blocs obtenus correspondent à la suite de la recherche, détermine que le transfert du contenu cible a été réalisé normalement, et dans le cas contraire, détermine que le transfert du contenu cible a été réalisé anormalement.

12. Programme pour amener une pluralité de processeurs à exécuter un procédé selon la revendication 11.
